# EUROPÄISCHE PATENTSCHRIFT

(11) **EP 3 651 831 B1**
(45) Veröffentlichungstag und Bekanntmachung des Hinweises auf die Patenterteilung: **28.02.2024**
(21) Anmeldenummer: 18740587.3
(22) Anmeldetag: 13.07.2018
(51) Int. Cl.: F04B 43/12, A61M 5/142, A61M 39/28

(54) **VORRICHTUNG ZUM STEUERN DES ÖFFNENS UND SCHLIESSENS EINES SCHLAUCHS**
DEVICE FOR CONTROLLING THE OPENING AND CLOSING OF A HOSE
DISPOSITIF DE COMMANDE DE L'OUVERTURE ET DE LA FERMETURE D'UN TUYAU

(30) Priorität: 14.07.2017 DE 102017115862
(43) Veröffentlichungstag der Anmeldung: 20.05.2020
(73) Patentinhaber: B. Braun Melsungen AG, 34212 Melsungen (DE)
(72) Erfinder: STEGER, Jürgen, 34327 Körle (DE); BREDE, Theresa, 34311 Naumburg (DE); MAURER, Artur, 34225 Baunatal-Großenritte (DE)
(74) Vertreter: Winter, Brandl - Partnerschaft mbB
(86) Internationale Anmeldenummer: PCT/EP2018/069086
(87) Internationale Veröffentlichungsnummer: WO 2019/012106

(56) Entgegenhaltungen:
- WO-A1-93/05829
- WO-A1-2011/121923
- WO-A1-2018/039018
- WO-A1-2018/223128
- JP-A- 2004 073 822
- US-A- 5 257 978

## Beschreibung

### Technischer Hintergrund

Die vorliegende Erfindung betrifft eine volumetrische Pumpe mit einer Schlauchklemmvorrichtung und einem Pumpengehäuse, an welchem eine Klappe oder Tür zum Verschließen eines Aufnahmeraums für einen flexiblen Schlauch angelenkt ist, in welchem eine Schlauchklemme angeordnet ist, die dafür ausgebildet ist, den darin eingelegten Schlauch bei geschlossener Klappe oder Tür freizugeben und bei geöffneter Klappe oder Tür dichtend abzuklemmen. Die Erfindung betrifft weiter ein Verfahren zum Steuern des Flusses durch einen flexiblen Schlauch in einer volumetrischen Pumpe mit einer solchen Schlauchklemmvorrichtung.

### Stand der Technik

Aus dem Stand der Technik sind diverse Infusionsschlauchpumpen bekannt. Meist handelt es sich dabei um Verdrängungspumpen die von außen her ein Volumen in einem flexiblen Schlauch (Einmalartikel) verdrängen und somit eine Kontamination der Pumpenmimik mit dem in dem Schlauch geführten Medium vermeiden. Auf dem Gebiet der Infusionstechnik ist es von immanenter Bedeutung, dass ein Schlauch, der eine Verbindung zwischen einem Patienten und einem Wirkstoffreservoir herstellt, niemals geöffnet sein darf, ohne dass die Durchflussrate kontrolliert wird, da dem Patienten ansonsten in einer sogenannten "Free-Flow-Situation" eine unkontrollierte Menge an Wirkstoff zugeführt werden könnte. Bei oben genannten Pumpen besteht deshalb die Notwendigkeit, den Fluss durch den Einmalartikel zu unterbrechen, wenn die Pumpe, bspw. zum Austausch des Einmalartikels, geöffnet wird. Hierzu wird üblicherweise dem Anwender vorgeschrieben (bspw. über eine Signalausgabe an einem Display der Infusionspumpe) vor dem Öffnen der Pumpe eine Sicherheitsklemme, bspw. eine Rollenklemme, manuell zu schließen. In dem Fall, dass der Anwender das Schließen der Sicherheitsklemme vergisst, entsteht eine Free-Flow-Situation, die den Patienten gefährdet.

Aus dem Stand der Technik ist die JP 2004 073822 A bekannt, die eine Infusionspumpe mit einer separaten Schlauchklemme offenbart, die in einen Klemmen-Aufnahmeschacht eingeschoben wird, wo sie von einem Vorspannmechanismus geschlossen wird.

WO 2011/121923 A1 offenbart eine Infusionspumpe mit einem Dreharm und einem Lösehebel.

US 5 257 978 A offenbart ein IV-Sicherheitsmodul, das mit einer peristaltischen IV-Pumpe verwendet wird, um den ungehinderten Fluss von IV-Flüssigkeit durch eine IV-Leitung zu verhindern, wenn die Tür der IV-Pumpe geöffnet ist.

WO 93/05829 A1 offenbart eine Vorrichtung zur Verwendung in einer peristaltischen Pumpe, die einen unbeabsichtigten Zustand des freien Flusses vor dem Laden oder nach der Entfernung einer Infusion verhindert.

WO 2018/223128 A1 offenbart eine Vorrichtung zum Erkennen und Entfernen einer Gasmasse aus einer Gefäßinfusionsleitung.

WO 2018/039018 A1 offenbart eine Vorrichtung zur Verhinderung des freien Flusses mit einem beweglichen Kolben.

### Kurzbeschreibung der Erfindung

Aufgabe der vorliegenden Erfindung ist es deshalb, Mittel und Wege bereitzustellen, den Fluss durch einen Schlauch derart zu steuern, dass das Entstehen einer Free-Flow-Situation vermieden werden kann.

Vorstehende Aufgabe wird durch eine Vorrichtung mit den Merkmalen des Anspruchs 1 sowie ein Verfahren mit den Merkmalen des Anspruchs 11 gelöst. Vorteilhafte Weiterbildungen sind Gegenstand der Unteransprüche.

Die Erfindung ist im beigefügten Anspruchssatz beschrieben. Grundsätzlich sieht die vorliegende Erfindung einer volumetrische Pumpe mit einer Schlauchklemmvorrichtung und einem Pumpengehäuse vor, an welchem eine Klappe oder Tür zum Öffnen/Verschließen eines Aufnahmeraums für einen flexiblen Schlauch angelenkt ist, in welchem eine Schlauchklemme angeordnet ist, die dafür ausgebildet ist, den darin eingelegten/einzulegenden Schlauch bei geschlossener Klappe oder Tür bzw. mit Schließen der Klappe oder Tür freizugeben und bei geöffneter Klappe oder Tür bzw. mit Öffnen der Klappe oder Tür dichtend abzuklemmen. Die Schlauchklemme weist ein am Pumpengehäuse beweglich gelagertes sowie in Abklemmrichtung federvorgespanntes Klemmschwert oder Klemmbacke auf, das an seinem einen Abschnitt eine mit einer pumpengehäusefesten Klemmbacke zusammenwirkende Klemmfläche/Klemmseite ausbildet, und das an seinem anderen Abschnitt eine Eingriffsfläche/Eingriffsseite ausbildet, an welcher ein an der Klappe oder Tür vorgesehener Vorsprung (bzw. Nocke/Steuerkurve) bei einem Schließvorgang derart abgleitet, dass hierbei eine Bewegungskraft auf das Klemmschwert entgegen der Federvorspannung aufgebracht wird. In anderen Worten handelt es sich um eine Kombination aus einem mittels einer Tür/Klappe verschließbaren Pumpengehäuse/Aufnahmeraum und einem Schlauch, der dazu vorgesehen ist, in dem Pumpengehäuse/Aufnahmeraum angeordnet zu werden. Dabei ist in dem Aufnahmeraum des Pumpengehäuses das relativbeweglich gelagerte Klemmschwert/Klemmbacke vorgesehen, welches erfindungsgemäß derart mit der gehäusefesten Klemmbacke und der Tür/Klappe bzw. dem daran vorgesehenen Vorsprung zusammenwirkt, dass es den Schlauch abhängig vom Öffnungszustand der Tür/Klappe öffnet oder schließt (abklemmt oder freigibt). Genauer gesagt, wirkt die Schlauchklemme derart mit der Tür/Klappe, der Klemmbacke und dem Schlauch zusammen, dass im geschlossenen Zustand der Tür ein Fluidfluss durch den Schlauch freigegeben ist und durch ein Öffnen der Tür/Klappe ein durch die Vorspannungsbeaufschlagung selbstbetätigtes (automatisches) klemmendes Verschließen des Schlauches stattfindet.

Besagtes Klemmschwert ist hierzu in Richtung der gehäusefesten Klemmbacke federvorgespannt und erfährt beim Schließen der Tür eine zwangsgeführte Auslenkung entgegen der Vorspannrichtung, weshalb sich die Klemmfläche des Klemmschwerts in Richtung weg von der gehäusefesten Klemmbacke bewegt.

Die erfindungsgemäße Vorrichtung vermeidet folglich das Entstehen einer Free-Flow-Situation, infolge einer vergessenen manuellen Schlauchabklemmung, da sie ein automatisches Abklemmen des Flusses durch den Schlauch bei einem Öffnen der Tür bereitstellt. Eine solche konstruktive Gestaltung mit gehäusefester Klemmbacke hat den Vorteil, dass zum einen die Klemme selbst keine zueinander relativbeweglichen Teile aufweisen muss, um eine klemmende Wirkung zu entfalten und daher vergleichsweise einfach aufgebaut sein kann und zum anderen bei einer solchen Gestaltung der Schlauch nicht durch die Klemme per se hindurch geführt werden muss sondern zwischen der Klemmfläche und der gehäusefesten Klemmbacke geführt werden kann. Im Stand der Technik werden dagegen oftmals schlauchseitige Klemmen mit durch den Klemmenkorpus geführtem Schlauch verwendet, die unter Umständen in das Pumpengehäuse einsetzbar sind, jedoch beim Austausch des als Einmalartikel ausgeführten Schlauches mit ausgetauscht werden müssen. Eine geräteseitige Klemme behebt diesen Nachteil, da lediglich der Schlauch austauscht wird, während die Klemme im/am Pumpengehäuse verbleiben kann.

Des Weiteren kann die gehäusefeste Klemmbacke an ihrer dem Klemmschwert zugewandten (Klemm-)Seite mit einer muldenförmigen Vertiefung oder Ausbuchtung ausgestaltet sein. Demzufolge kann der Anwender den Schlauch in der muldenartigen Ausbuchtung einlegen bzw. quasi vorfixieren, bevor das Klemmschwert beim späteren (erneuten) Öffnen der Tür gegen den eingelegten Schlauch drückt. Die Öffnung ist hierbei so ausgelegt, dass der Schlauch "leicht" ohne Kraftanstrengung in die Ausbuchtung eingekippt werden kann. Der Schlauch muss also nicht unter das mit großer Kraft belastete Schwert geschoben werden.

Der Hinterschnitt der Ausbuchtung sorgt ferner dafür, dass der Schlauch nicht mehr aus der Ausbuchtung heraus fällt bzw. von der gehäusefesten Klemmbacke abgleitet.

Ferner wird mit Schließen der Tür das Klemmschwert von der an der Tür ausgebildeten Kontur (Vorsprung) so angehoben, dass sich der Schlauch leicht unter das Klemmschwert (bzw. in den Spalt zwischen Klemmschwert und gehäusefester Klemmbacke) schieben lässt, ohne dabei beschädigt zu werden.

Gemäß einem Aspekt der Erfindung kann der Vorsprung, an welchem die Eingriffsfläche des Klemmschwerts abgleitet, sich im Wesentlichen in Schließrichtung der Tür erstrecken und beim Verschließen der Tür die Eingriffsfläche des Klemmschwerts untergreifen und nach Art einer Rampe mit zunehmender Schließbewegung das Klemmschwert kontinuierlich verschwenken.

Gemäß einer bevorzugten Ausführungsform der Erfindung kann das Klemmschwert, insbesondere an einem Endabschnitt desselben, über eine Klemmenachse drehbar am Pumpengehäuse gelagert sein. Die vorspannkraftbeaufschlagte Abklemmbewegung des Klemmschwerts ist in einem solchen Fall also eine Schwenkbewegung in Richtung zu der gehäusefesten Klemmbacke. Alternativ kann das Klemmschwert auch bspw. über eine Kulissenführung oder dergleichen Linearführung relativbeweglich am Pumpengehäuse gelagert sein. Ein drehbares Lagern des Klemmschwerts am Gehäuse mittels einer Klemmenachse kann gegenüber anderen Lagerungsalternativen Fertigungsaufwand einsparen.

Gemäß einem bevorzugten Aspekt können die Klemmfläche und/oder die Eingriffsfläche an zumindest einem Endabschnitt des Klemmschwerts angeordnet sein. Bevorzugt können die Klemmfläche und/oder die Eingriffsfläche an zumindest einem der Klemmenachse gegenüberliegenden Endabschnitt angeordnet sein. Weiter bevorzugt können die Klemmfläche und/oder die Eingriffsfläche an zumindest einem sich schenkelförmig (strahlenförmig) von der Klemmenachse weg erstreckenden Endabschnitt, des Klemmschwerts angeordnet sein. Besonders bevorzugt können die Klemmfläche und die Eingriffsfläche separat an zwei sich schenkelförmig (strahlenförmig) von der Klemmenachse weg erstreckenden Endabschnitten, des Klemmschwerts angeordnet sein.

Eine bevorzugte Konstruktion des Klemmschwerts sieht dessen Ausbildung nach Art einer Nocke oder eines Schwenkhebels vor mit einer maulartigen Längseinkerbung (Hebellängsrichtung in Richtung zur Schwenkachse) unter Ausbildung eines Ober- und Unterkiefers. Der Oberkiefer definiert dabei die Klemmfläche, welche sich zum Unterkiefer hin zuwendet (obere Innenseite der Längseinkerbung), wohingegen der Unterkiefer die Eingriffsfläche definiert, welche sich an einer Unterseite (zur Längseinkerbung abgewandte Seite) des Unterkiefers ausbildet. Die gehäusefeste Klemmbacke ist dabei innerhalb der Längseinkerbung positioniert und wirkt somit in Zusammenwirken mit dem Unterkiefer als eine Art Schwenkbegrenzung zumindest in Öffnungsrichtung der Klemmschwerts unter Ausbildung eines weitedefinierten Öffnungsspalts zwischen der oberkieferseitigen Klemmfläche und der gehäusefesten Klemmbacke.

Gemäß einer vorteilhaften Weiterbildung der Erfindung kann die Federvorspannung derart ausgelegt sein, dass der flexible Schlauch infolge der Federvorspannung zwischen der Klemmfläche des Klemmschwerts und der Klemmbacke des Gehäuses unter Überwindung seiner internen Elastizität dichtend abgeklemmt wird.

Gemäß einem weiter bevorzugten Aspekt der Erfindung kann die Klemme durch ein Vorspannelement in Abklemmrichtung vorgespannt sein. Bevorzugt kann das Vorspannelement als Druck-, Zug oder Drehfeder ausgebildet sein und die Klemme in Abklemmrichtung (in der Schließposition) vorgespannt halten. Es ist ebenfalls angedacht, dass das Vorspannelement als integraler Abschnitt der Klemme deren material eigene Elastizität ausnutzt. Gemäß einer besonders bevorzugten Ausführungsform der Erfindung kann die Schlauchklemmvorrichtung ein als axiale Druckfeder, insbesondere als Spiral- oder Wellenfeder, ausgebildetes Vorspannelement aufweisen, welches gehäusefest gelagert ist und an einem Federangriffspunkt die Vorspannkraft in Abklemmrichtung auf das Klemmschwert aufbringt. Vorteilhafterweise können Vorspannelement und Federangriffspunkt derart angeordnet sein, dass der Federangriffspunkt im Wesentlichen auf Höhe der Klemmfläche liegt und/oder die Vorspannkraft im Wesentlichen senkrecht zur Klemmfläche wirkt. Auf diese Weise lässt sich eine effiziente Krafteinleitung sicherstellen.

Gemäß einem bevorzugten Aspekt kann der klappenseitige Vorsprung (Nocke/Steuerkurve), an welchem die Eingriffsfläche (bevorzugt ein freies Ende eines schenkelförmigen Endabschnitts) des Klemmschwerts abgleitet, als eine bogen- oder teilkreisförmige Führungskontur/Platte ausgebildet sein, an welcher die Eingriffsfläche bei einem Schließen der Tür oder Klappe derart abgleitet, dass ein zwangsgeführtes Anheben des Klemmschwerts und somit ein Freigeben des flexiblen Schlauchs bewirkt wird. Besonders bevorzugt ist der Vorsprung (die Führungskontur) derart ausgebildet, dass das Klemmschwert in der geschlossenen Position der Tür (entgegen der Vorspannkraft des Vorspannelements) in einer definierten Öffnungsposition gehalten wird (mit der Eingriffsfläche auf dem Vorsprung / der Führungskontur aufliegt. Es besteht aber auch die Möglichkeit, dem Vorsprung eine Eigenelastizität zuzuweisen, mit einer Federkraft (bzw. Federsteifigkeit) größer als die der Vorspannfeder, sodass das Klemmschwert beim Schließen der Klappe/Tür gegen die Vorspannfeder verschwenkt/bewegt wird, bis der Unterkiefer gegen die gehäusefeste Klemmbacke anschlägt.

Gemäß einer weiter bevorzugten Ausführungsform der Erfindung kann die Schlauchklemmvorrichtung in anderen Worten ausgedrückt einen Einlegeabschnitt aufweisen, welcher durch einen zur Klemmbacke benachbarten Gehäuseabschnitt und einen zur Klemmfläche benachbarten Klemmschwertabschnitt gebildet wird, sodass ein flexibler Schlauch darin in zur Klemmfläche benachbarter Position zwischen Gehäuse und Klemmschwert einlegbar ist und welcher konstruktiv derart abgestimmt ist, dass ein darin eingelegter flexibler Schlauch in einem zum Durchfluss freigegebenen Zustand formschlüssig und/oder vorspannkraftbeaufschlagt gehalten wird. Mit anderen Worten kann ein freies (distales bzw. der Pumpengehäuseaußenseite zugewandtes) Ende des Klemmschwerts zusammen mit einem zur gehäusefesten Klemmbacke benachbarten Pumpengehäuseabschnitt einen definierten Einlegeabschnitt bilden. Vorzugsweise können die beiden Abschnitte derart zusammenwirken, dass ein zwischen diesen eingelegter Schlauch form- und/oder kraftschlüssig gehalten und gleichzeitig für einen Durchfluss geöffnet/freigegeben ist. Auf diese Weise kann eine definierte Einlegeposition geschaffen werden. Dazu kann der Einlegeabschnitt aus zwei komplementären rinnenförmigen Abschnitten des Pumpengehäuses und des Klemmschwerts ausgebildet sein.

Gemäß einer vorteilhaften Weiterbildung der Erfindung kann die Tür oder Klappe den vorsprung- oder rippenartigen Einschubabschnitt aufweisen, welcher bei einem Verschließen der Tür oder Klappe einen im Einlegeabschnitt eingelegten flexiblen Schlauch zwischen die Klemmbacke und die Klemmfläche schiebt (d.h. in die Längskerbe hinein). In anderen Worten kann die Tür einen Einschubabschnitt ausbilden, welcher bei einem Verschließen der Tür den Schlauch aus einer ersten (Einlege-)Position in eine zweite (Abklemm-)Position verschiebt. Eine solche Umpositionierung des Schlauches infolge einer (Schließ-)Bewegung der Tür ermöglicht eine differenzierte Steuerung des Flusses durch den Schlauch mit rein mechanischen Mitteln, da Pumpengehäuse, Tür und Klemme an den beiden verschiedenen vorgesehenen Schlauchpositionen jeweils unterschiedlich zusammenwirken können.

Gemäß einem bevorzugten Aspekt kann das Pumpengehäuse, insbesondere in einem unteren Aufnahmeraumrandabschnitt, einen Anschlag ausbilden, gegen welchen das Klemmschwert durch die Vorspannkraft gehalten/gedrängt wird und an welchem ein Anschlagsabschnitt des Klemmschwerts anschlägt, um einen vorbestimmten Spalt zwischen Klemmfläche und Klemmbacke zu definieren. Anders ausgedrückt kann durch den Anschlag in der Schließposition ein definierter Spalt/Abstand zwischen der Klemmfläche des Klemmschwerts und der gehäusefesten Klemmbacke erzeugt werden, wobei der Spalt vorzugsweise so definiert sein kann, dass ein zwischen Klemmschwert und Klemmfläche angeordneter Schlauch zuverlässig (fluiddicht) verschlossen wird, ohne dass es dabei zu einer Beschädigung des Schlauches kommt. In anderen Worten, können Anschlag, Anschlagsabschnitt, Klemmschwert und gehäusefeste Klemmbacke derart konstruktiv aufeinander abgestimmt sein, dass der definierte (Klemm-)Spalt entsteht.

Gemäß einer weiter bevorzugten Ausführungsform kann die Klemme einstückig, insbesondere aus Kunststoff, ausgebildet sein. Klemmfläche und Eingriffsfläche (bzw. die schenkelförmigen Endabschnitte, an welchen diese angeordnet sind) können bevorzugt integral mit dem Klemmenkorpus gefertigt sein. Dies ermöglicht eine besonders einfache Konstruktion und Fertigung der Klemme, bspw. im Spritzgussverfahren.

Ferner betrifft die Erfindung ein Verfahren zur Steuerung des Flusses durch einen Schlauch, der an oder in einem mittels einer Tür oder Klappe verschließbaren Pumpengehäuse angeordnet ist, an welchem ein Klemmschwert relativbeweglich angelenkt ist, das dazu ausgebildet ist, mit einer pumpengehäusefesten Klemmbacke zusammenzuwirken, um den Schlauch dichtend abzuklemmen, insbesondere mittels einer Vorrichtung gemäß einem der vorgenannten Aspekte.

Erfindungsgemäß weist das Verfahren zumindest die folgenden Schritte auf:
- Einlegen des Schlauches an einer vorbestimmten Einlegeposition an oder in dem Pumpengehäuse;
- Verschließen des Pumpengehäuses mittels der Tür und gleichzeitiges zwangsgeführtes Bewegen der Klemme und/oder des Schlauches infolge der Bewegung der Tür.

Weiter bevorzugt kann oben genanntes Verfahren die folgenden Schritte aufweisen:
- Einlegen des Schlauches an einer vorbestimmten Einlegeposition an oder in dem Pumpengehäuse;
- Verschließen des Pumpengehäuses mittels der Tür und gleichzeitiges zwangsgeführtes Bewegen der Klemme in die Öffnungsposition und Schlauches auf die Klemmfläche infolge der Bewegung der Tür
- Öffnen der Tür und gleichzeitiges vorspannkraftbeaufschlagtes Bewegen der Klemme in die Schließposition.

### Kurzzusammenfassung der Figuren

Die Erfindung wird nachstehend anhand bevorzugter Ausführungsbeispiele unter Bezugnahme auf die begleitenden Figuren näher erläutert. Die Figuren sind lediglich schematischer Natur und dienen ausschließlich dem Verständnis der Erfindung. Die gleichen Elemente sind mit denselben Bezugszeichen versehen.

Es zeigen:
Fig. 1 eine schematische Darstellung einer Vorrichtung gemäß einer bevorzugten Ausführungsform der Erfindung, nach dem Einlegen eines Einmalartikels;
Fig. 2 eine schematische Darstellung der Vorrichtung gemäß der bevorzugten Ausführungsform der Erfindung, nach dem Verschließen der Tür; und
Fig. 3 eine schematische Darstellung der Vorrichtung gemäß der bevorzugten Ausführungsform der Erfindung, nach dem Öffnen der Tür.

### Figurenbeschreibung

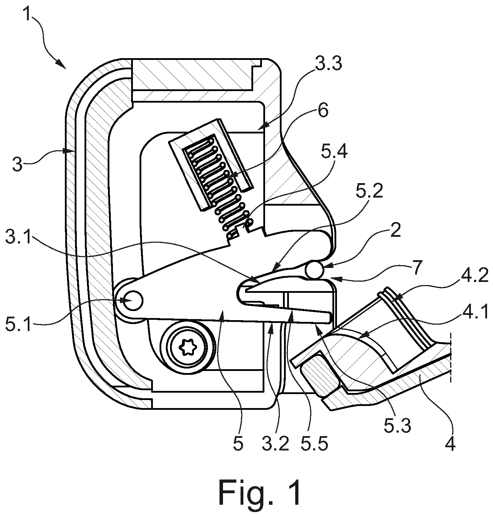

Fig. 1 zeigt eine bevorzugte Ausführungsform einer erfindungsgemäßen Schlauchklemmvorrichtung 1 zum Steuern des Flusses durch einen flexiblen Schlauch 2 in einer schematischen Ansicht. Im dargestellten Beispiel ist die Vorrichtung 1 in eine volumetrische Infusionspumpe integriert. Diese weist grundsätzlich ein Pumpengehäuse 3 auf, welches durch eine schwenkbare Tür 4 verschließbar ist. Zum Betreiben der Infusionspumpe, wird der flexible Schlauch 2, welcher als Einmalartikel ausgeführt ist und eine Fluidverbindung zwischen einem Patienten und einem Wirkmittelreservoir herstellt, zwischen Pumpengehäuse 3 und Tür 4 eingelegt. Die Infusionspumpe weist flussaufwärts (in Richtung des Wirkmittelreservoirs) der dargestellten Schlauchklemmvorrichtung eine im Pumpengehäuse 3 angeordnete (nicht dargestellte) Schieberperistaltik auf, welche, im Zusammenspiel mit einer als Gegenlager fungierenden Fläche der Tür 4, eine Volumenverdrängung in dem dazwischen positionierten flexiblen Schlauch 2 und damit eine definierte Förderung des Wirkmittels aus dem Wirkmittelreservoir bereitstellt.

Wird die Tür 4, bspw. zum Austauschen des Schlauches 2, geöffnet, so steht sie nicht mehr als Gegenlager zur Peristaltik zur Verfügung und es kann eine unkontrollierte Free-Flow-Situation im Schlauch 2 entstehen. Um dies zu verhindern, weist die dargestellte Schlauchklemmvorrichtung 1 eine Klemme 5 auf, welche dazu ausgebildet ist, den Schlauch 2 in einem solchen Fall (bei einem Öffnen der Tür 4) rein mechanisch aktuiert selbstständig abzuklemmen (klemmend zu verschließen). Durch eine solche rein mechanische Lösung kann gegenüber den im Stand der Technik bekannten elektronisch gesteuerten Schlauchklemmvorrichtung der Fertigungsaufwand und die Fehleranfälligkeit reduziert werden. Die dargestellte Vorrichtung 1 hat zudem den Vorteil, dass sie eine Geräteseitig fest installierte, wiederverwendbare Klemme 5 bereitstellt, welche zum Abklemmen des Schlauches 2 mit einer gehäusefesten Klemmbacke 3.1 zusammenwirkt wohingegen im Stand der Technik oftmals beim Austauschen des Schlauches auch die (schlauchseitige) Klemme ausgetauscht werden muss.

Die Klemme 5 hat in dem dargestellten Beispiel ein Klemmschwert 5, das in einer Pumpengehäuseöffnung angeordnet ist, welche einen durch die Tür 4 verschließbaren Aufnahmeraum 3.3 für den flexiblen Schlauch 2 bildet. Das Klemmschwert 5 ist über eine Klemmenachse 5.1 relativdrehbar am Pumpengehäuse 3 bzw. in der Pumpengehäuseöffnung gelagert (bspw. über ein Gleit- oder Wälzlager). Ein Vorspannelement 6, hier eine vorbestimmt gestauchte lineare Spiralfeder, welches ebenfalls am Pumpengehäuse 3 gelagert ist und das Klemmschwert 5 an einem Federangriffspunkt 5.4 angreift, spannt das Klemmschwert 5 in Richtung einer vordefinierten Schließposition vor. In der Schließposition wirkt eine Klemmfläche 5.2 des Klemmschwerts 5 mit einer Klemmbacke 3.1 des Pumpengehäuses zusammen, um einen dazwischen positionierten flexiblen Schlauch 2 abzuklemmen. Man könnte also auch sagen, das Klemmschwert 5 ist in Abklemmrichtung vorgespannt. Die Klemmfläche 5.2 ist im dargestellten Beispiel an einem ersten Schenkel oder Ausleger (Endabschnitt) des Klemmschwerts 5 angeordnet. Der Federangriffspunkt 5.4 ist vorteilhafterweise so angeordnet, dass er (in Schenkellängsrichtung) auf Höhe der Klemmfläche 5.2 liegt und ein gedachter Vorspannkraftvektor die Klemmfläche 5.2, insbesondere im Wesentlichen senkrecht, schneidet.

Das Klemmschwert 5 liegt in der Schließposition mit einem Anschlagsabschnitt 5.5 auf einem durch das Pumpengehäuse 3 gebildeten Anschlag 3.2 auf bzw. wird durch das Vorspannelement 6 gegen den Anschlag 3.2 vorgespannt. Auf diese Weise wird ein definierter Klemmspalt zwischen Klemmfläche 5.2 und Klemmbacke 3.1 erzeugt, welcher ein zuverlässiges, fluiddichtes Verschließen des Schlauches 2 sicherstellt, zugleich aber gewährleistet, dass der Schlauch 2 nicht beschädigt wird. Der Anschlag 3.2 wird in dem dargestellten Beispiel durch einen unteren Rand des Schlauchaufnahmeraums 3.3 gebildet, in welchem das Klemmschwert 5 angeordnet ist, wodurch die bestehende Geometrie zum Erfüllen dieser Funktion genutzt wird.

Der Anschlagsabschnitt 5.5 des Klemmschwerts 5 ist im dargestellten Beispiel an einem zweiten Schenkel oder Auslegerarm desselben angeordnet, welcher (von der Klemmenachse 5.1 aus gesehen) über den Anschlag 3.2 (zur Pumpengehäuseöffnung hin) hinausragt und somit als Hebel zum (manuellen) Auslenken des Klemmschwerts 5 entgegen der Vorspannrichtung des Vorspannelements 6 genutzt werden kann. In der geöffneten Position der Tür 4, ist das (distale) freie Ende des zweiten Schenkels des Klemmschwerts 5, an welchem der Anschlagsabschnitt 5.5 angeordnet ist, freigegeben und dieser liegt aufgrund der Kraftbeaufschlagung durch das Vorspannelement 6 auf dem durch das Pumpengehäuse 3 gebildeten Anschlag 3.2 auf (vgl. Fig. 1).

Die Tür 4 weist einen als teilkreisförmige Führungskontur ausgebildeten Vorsprung 4.1 auf, welcher bei einem Verschließen der Tür 4 das (distalen) mit einer am freie Ende des zweiten Schenkels des Klemmschwerts 5 angeordneten Eingriffsfläche 5.3 zusammenwirkt, um das Klemmschwert 5 in einer zwangsgeführt abgleitenden Bewegung entgegen der Vorspannrichtung auszulenken und somit das gesamte Klemmschwert 5 und insbesondere die Klemmfläche 5.2 von der Klemmbacke 3.1 abzuheben. In der geschlossenen Position der Tür 4, hält der Vorsprung 4.1 das Klemmschwert 5 in einer vordefinierten Öffnungsposition, in welcher Klemmfläche 5.2 und Klemmbacke 3.2 derart beabstandet sind, dass ein definierter Durchfluss durch einen zwischen diesen beiden Komponenten positionierten Schlauch 2 möglich ist.

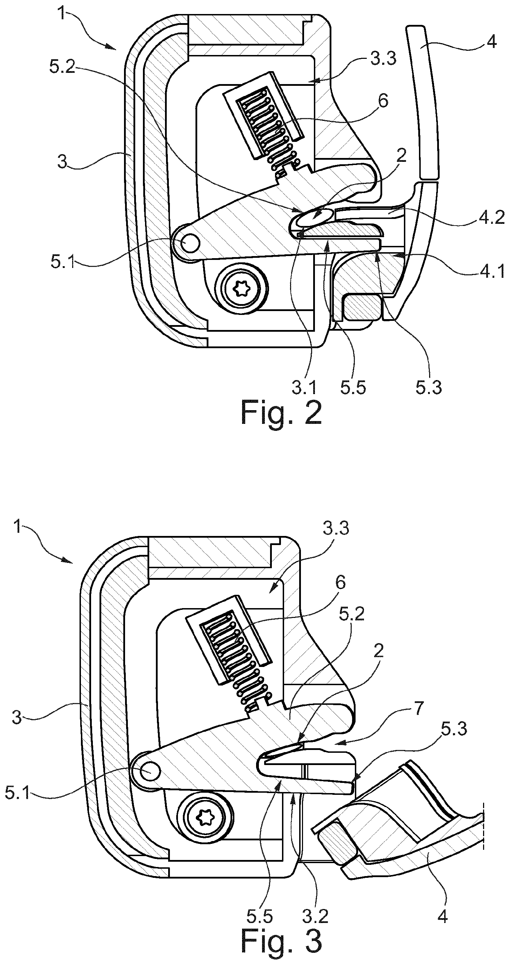

Das (distale) freie Ende des ersten Schenkels des Klemmschwerts 5, an welchem die Klemmfläche 5.2 angeordnet ist, bildet zusammen mit einem zur Klemmbacke 3.1 benachbarten Abschnitt des Pumpengehäuses 3 einen definierten Einlegeabschnitt 7 und somit eine definierte Einlegeposition für den Schlauch 2. Genauer gesagt bilden das (distale) freie Ende des ersten Schenkels (an welchem die Klemmfläche 5.2 angeordnet ist) und der oben genannte Pumpengehäuseabschnitt 7 zwei einander zugewandte rinnenförmige Flächen (vgl. Fig. 3) aus, die dazu ausgebildet sind, den Schlauch 2 teilweise komplementär zu umgreifen und somit zu halten, ohne dabei eine klemmende Wirkung zu entfalten, wie bspw. Fig. 1 zu entnehmen ist. Hierzu sind die beiden rinnenförmigen Flächen in der (durch den Anschlag 3.2 definierten) Schließposition der Klemme 5 in einem definierten Abstand zueinander beabstandet.

Die Tür 4 weist weiter einen hier (horizontal-)rippenförmig ausgebildeten Einschubabschnitt oder -vorsprung 4.2 auf, der bei einem Verschließen der Tür 4 den Schlauch 2 aus dem Einlegeabschnitt 7 heraus und auf die gehäusefeste Klemmbacke 3.1 schiebt. Die Steuerkurve 4.1 bewegt zugleich das Klemmschwert 5 aus der Schließposition in die Öffnungsposition, sodass der Schlauch 2 unter die Klemmfläche 5.2 und auf die Klemmbacke 3.1 geschoben werden kann (vgl. Fig. 2).

Wird nun die Tür 4 wieder geöffnet, so wird die Eingriffsfläche 5.3 des Klemmschwerts 5 bzw. das freie Ende des zweiten Schenkels wieder freigegeben und bewegt sich infolge der Vorspannungskraft des Vorspannelements 6 wieder in die Schließposition. Da der Schlauch 2 beim Verschließen der Tür 4 auf die Klemmbacke 3.1 des Pumpengehäuses 3 geschoben wurde, wird der Schlauch 2 nun im geöffneten Zustand der Tür 4 durch das in Abklemmrichtung vorgespannte Klemmschwert 5 selbstbetätigt abgeklemmt und stellt auf diese Weise die Free-Flow-Sicherung bereit. Die Vorspannkraft des Vorspannelements 6 ist derart eingestellt, dass sie die Eigenelastizität des Schlauchs 2 zum Verschließen desselben überwindet.

Das Klemmschwert 5 an sich ist im dargestellten Ausführungsbeispiel als einstückiges Bauteil mit zwei sich von der Klemmenachse 5.1 weg und zur Tür 4 hin erstreckenden Schenkeln/Auslegern ausgeführt, an welchen die Klemmfläche 5.2, die Eingriffsfläche 5.3 und der Anschlagsabschnitt 5.5 integral ausgebildet und angeordnet sind.

Die beiden Schenkel nehmen vorteilhafterweise die gehäusefest ausgebildete Klemmbacke 3.1 zwischen sich auf, wie den Figuren zu entnehmen ist. Besagte Klemmbacke 3.1 ist im dargestellten Beispiel als Abschnitt einer horizontal vorspringende Rippe im Eingangsbereich der Pumpengehäuseöffnung ausgebildet, wobei in Richtung hin zur Tür 4 (dem Pumpengehäuseäußeren) benachbart zur Klemmbacke 3.1 der rinnenförmige Einlegeabschnitt 7 an der Rippe angeordnet ist.

Zusammenfassend kann ein Bedienvorgang der Erfindungsgemäßen Vorrichtung 1 durch einen Anwender bspw. wie folgt ablaufen:
Die Tür 4 des Pumpengehäuses 3 ist zunächst geöffnet und das Klemmschwert 5 liegt, durch das Vorspannelement 6 vorgespannt, mit dem Anschlagsabschnitt 5.5 auf dem Anschlag 3.2 auf und befindet sich somit in der Schließposition. In diesem Zustand wird (wie in Fig. 1 gezeigt) ein Schlauch 2 in den Einlegeabschnitt 7, der durch Pumpengehäuse 3 und Klemmschwert 5 definiert wird eingelegt. Der Anschlag 3.2 definiert eine Öffnung des Einlegeabschnitts 7 so, dass der Schlauch 2 fest gehalten jedoch nicht abgeklemmt/verschlossen wird.

Wird nun, wie in Fig. 2 dargestellt, die Tür zu Beginn einer Behandlung (Infusion) geschlossen, so bewegt die Führungskontur 4.1 das Klemmschwert 5 entgegen der Vorspannkraft aus der Schließposition in die Öffnungsposition, die dadurch definiert wird, dass das die Eingriffsfläche 5.3 (hier: das freie Ende des zweiten Schenkels) des Klemmschwerts 5 auf der Steuerkurve 4.1 vorgespannt aufliegt. Gleichzeitig schiebt der Einschubabschnitt 4.2 der Tür 4 den Schlauch 2 von dem Einlegeabschnitt 7 aus nach innen auf die benachbarte Klemmbacke 3.1 und unter das in der Öffnungsposition gehaltene Klemmschwert 5. Auf diese Weise wird in der geschlossenen Position der Tür 4 der Durchfluss durch den Schlauch 2 ermöglicht.

Schließlich wird nach Ende der Behandlung die Tür 4 geöffnet, um den Schlauch 2 entnehmen zu können. Hierbei wird die Eingriffsfläche 5.3 (das freie Ende des zweiten Schenkels) von der Steuerkurve 4.1 freigegeben, so dass die Klemme unter der Vorspannkraftbeaufschlagung durch das Vorspannelement 6 in die Schließposition zurückkehrt und den nun zwischen Klemmfläche 5.2 und Klemmbacke 3.1 positionierten Schlauch 2 fluiddicht abklemmt, um eine Free-Flow-Situation zu vermeiden.
- 1: Schlauchklemmvorrichtung;
- 2: Schlauch/Einmalartikel;
- 3: Pumpengehäuse;
- 3.1: Klemmbacke;
- 3.2: Anschlag;
- 3.3: Aufnahmeraum;
- 4: Tür/Klappe;
- 4.1: Vorsprung/Führungskontur/Steuerkurve;
- 4.2: Einschubabschnitt;
- 5: Schlauchklemme/Klemmschwert;
- 5.1: Klemmenachse;
- 5.2: Klemmfläche;
- 5.3: Eingriffsfläche/freies Ende des zweiten Schenkels;
- 5.4: Federangriffspunkt;
- 5.5: Anschlagsabschnitt;
- 6: Vorspannelement/ axiale Druckfeder; und
- 7: Einlegeabschnitt.

## Patentansprüche

1. Volumetrische Pumpe mit einer Schlauchklemmvorrichtung (1) und
einem Pumpengehäuse (3), an welchem eine Klappe oder Tür (4) zum Verschließen eines Aufnahmeraums für einen flexiblen Schlauch (2) angelenkt ist, in welchem eine Schlauchklemme (5) angeordnet ist, die dafür ausgebildet ist, den darin eingelegten Schlauch (2) bei geschlossener Klappe oder Tür (4) freizugeben und bei geöffneter Klappe oder Tür (4) dichtend abzuklemmen,
**dadurch gekennzeichnet, dass**
die Schlauchklemme (5) ein am Pumpengehäuse (3) beweglich gelagertes sowie in Abklemmrichtung federvorgespanntes Klemmschwert (5) hat, das an einem Abschnitt eine mit einer pumpengehäusefesten Klemmbacke (3.1) zusammenwirkende Klemmfläche (5.2) ausbildet, und das an einem anderen Abschnitt eine Eingriffsfläche (5.3) ausbildet, an welcher ein an der Klappe oder Tür (4) vorgesehener Vorsprung (4.1) bei einem Schließvorgang durch eine Schwenkbewegung der Klappe oder Tür (4) derart abgleitet, dass hierbei eine Bewegungskraft auf das Klemmschwert (5) entgegen der Federvorspannung aufgebracht wird.

2. Volumetrische Pumpe nach Anspruch 1 **dadurch gekennzeichnet, dass** das Klemmschwert (5), insbesondere an einem Endabschnitt desselben, über eine Klemmenachse (5.1) drehbar am Pumpengehäuse (3) gelagert ist.

3. Volumetrische Pumpe nach Anspruch 1 oder 2, **dadurch gekennzeichnet, dass** die Klemmfläche (5.2) und/oder die Eingriffsfläche (5.3) an zumindest einem Endabschnitt, bevorzugt an zumindest einem der Klemmenachse (5.1) gegenüberliegenden Endabschnitt, besonders bevorzugt an zwei sich schenkelförmig von der Klemmenachse (5.1) weg erstreckenden Endabschnitten, des Klemmschwerts (5) angeordnet sind.

4. Volumetrische Pumpe nach einem der Ansprüche 1 bis 3, **dadurch gekennzeichnet, dass** die Federvorspannung derart ausgelegt ist, dass ein zwischen Klemmfläche (5.2) des Klemmschwerts (5) und Klemmbacke (3.1) des Gehäuses (3) eingelegter flexible Schlauch (2) bei geöffneter Klappe oder Tür (4) infolge der Federvorspannung unter Überwindung seiner internen Elastizität dichtend abgeklemmt wird.

5. Volumetrische Pumpe nach einem der vorgenannten Ansprüche,
**gekennzeichnet durch** ein als axiale Druckfeder, insbesondere als Spiralfeder, ausgebildetes Vorspannelement (6), welches gehäusefest gelagert ist und an einem Federangriffspunkt (5.4) die Vorspannkraft in Abklemmrichtung auf das Klemmschwert (5) aufbringt.

6. Volumetrische Pumpe nach einem der vorgenannten Ansprüche, **dadurch gekennzeichnet, dass** der Vorsprung (4.1), an welchem die Eingriffsfläche (5.3) des Klemmschwerts (5) abgleitet, sich im Wesentlichen in Schließrichtung der Tür (4) erstreckt, beim Verschließen der Tür (4) die Eingriffsfläche (5.3) des Klemmschwerts (5) untergreift und nach Art einer Rampe mit zunehmender Schließbewegung das Klemmschwert (5) kontinuierlich verschwenkt.

7. Volumetrische Pumpe nach Anspruch 6, **dadurch gekennzeichnet, dass** der Vorsprung (4.1), an welchem die Eingriffsfläche (5.3) des Klemmschwerts (5) abgleitet als eine bogen- oder teilkreisförmige Führungskontur ausgebildet ist, an welcher die Eingriffsfläche (5.3) bei einem Schließen der Klappe oder Tür (4) derart abgleitet, dass ein zwangsgeführtes Anheben des Klemmschwerts (5) und somit ein Freigeben des flexiblen Schlauchs (2) bewirkt wird.

8. Volumetrische Pumpe nach einem der vorgenannten Ansprüche,
**gekennzeichnet durch** einen Einlegeabschnitt (7), welcher durch einen zur Klemmbacke (3.1) benachbarten Gehäuseabschnitt und einen zur Klemmfläche (5.2) benachbarten Klemmenabschnitt gebildet wird, sodass ein flexibler Schlauch (2) darin in zur Klemmfläche (5.2) benachbarter Position zwischen Gehäuse (3) und Klemmschwert (5) einlegbar ist und welcher konstruktiv derart abgestimmt ist, dass ein darin eingelegter flexibler Schlauch (2) in einem zum Durchfluss freigegebenen Zustand formschlüssig und/oder vorspannkraftbeaufschlagt gehalten wird.

9. Volumetrische Pumpe nach Anspruch 8, **dadurch gekennzeichnet, dass** die Tür oder Klappe (4) einen vorsprung- oder rippenartigen Einschubabschnitt (4.2) aufweist, welcher bei einem Verschließen der Tür oder Klappe (4) einen im Einlegeabschnitt (7) eingelegten flexiblen Schlauch (2) zwischen die Klemmbacke (3.1) und die Klemmfläche (5.2) schiebt.

10. Volumetrische Pumpe nach einem der vorgenannten Ansprüche, **dadurch gekennzeichnet, dass** das Pumpengehäuse (3), insbesondere in einem unteren Randabschnitt des Aufnahmeraums, einen Anschlag (3.2) ausbildet, gegen welchen das Klemmschwert (5) bei geöffneter Klappe oder Tür (4) mit einem Anschlagsabschnitt (5.5) vorspannkraftbeaufschlagt aufliegt, und welcher konstruktiv derart mit dem Klemmschwert (5) und der Klemmbacke (3.1) abgestimmt ist, dass er bei aufliegendem Klemmschwert (5) einen Spalt zwischen Klemmfläche (5.2) und Klemmbacke (3.1) definiert.

11. Verfahren zur Steuerung des Flusses durch einen Schlauch (2), der an oder in einem mittels einer Tür oder Klappe (4) verschließbaren Pumpengehäuse (3) einer volumetrischen Pumpe nach einem der vorstehenden Ansprüche angeordnet ist, an welchem ein Klemmschwert (5) relativbeweglich angelenkt ist, das dazu ausgebildet ist, mit einer pumpengehäusefesten Klemmbacke (3.1) zusammenzuwirken, um den Schlauch (2) dichtend abzuklemmen,
**gekennzeichnet durch** die Schritte:
- Einlegen des Schlauches (2) an einer vorbestimmten Position an oder in dem Pumpengehäuse (3);
- Verschließen des Pumpengehäuses (3) mittels der Tür (4) und gleichzeitiges zwangsgeführtes Bewegen der Schlauchklemme (5) in die Öffnungsposition und des Schlauches (2) auf eine Klemmfläche der Klemme infolge der Bewegung der Tür (4);
- Öffnen der Tür (4) und gleichzeitiges selbstbetätigtes Verschließen des Schlauches (2) durch die Schlauchklemme (5).

## Claims

1. A volumetric pump comprising a clamping device (1) and
a pump casing (3) to which a flap or door (4) for closing an accommodating chamber for a flexible tube (2) is articulated, in which accommodating chamber a tube clamp (5) is arranged that is configured to release the tube (2) inserted therein while the flap or door (4) is closed and to pinch off the tube (2) in a sealing manner when the flap or door (4) is opened,
**characterized in that**
the tube clamp (5) includes a clamping blade (5) being movably supported on the pump casing (3) and being spring-pretensioned in the pinching direction, wherein the clamping blade at one portion forms a clamping surface (5.2) interacting with a clamping jaw (3.1) fixed to the pump casing and at another portion forms an engaging surface (5.3) of which a projection (4.1) provided on the flap or door (4) slips off during a closing operation by a pivoting movement of the flap or door (4) so that accordingly a force of movement is applied to the clamping blade (5) against the spring pretension.

2. The volumetric pump according to claim 1, **characterized in that** the clamping blade (5) is rotatably supported, in particular at an end portion thereof, on the pump casing (3) via a clamp axle (5.1).

3. The volumetric pump according to claim 1 or 2, **characterized in that** the clamping surface (5.2) and/or the engaging surface (5.3) is/are arranged on at least one end portion, preferably on at least one end portion opposite to the clamp axle (5.1), especially preferred on two end portions extending in leg shape away from the clamp axle (5.1), of the clamping blade (5).

4. The volumetric pump according to one of the claims 1 to 3, **characterized in that** the spring pretension is designed in such manner that a flexible tube (2) inserted between the clamping surface (5.2) of the clamping blade (5) and the clamping jaw (3.1) of the casing (3) is pinched off in a sealing manner, when the flap or door (4) is opened, due to the spring pretension while overcoming its internal elasticity.

5. The volumetric pump according to one of the afore-mentioned claims, **characterized by** a biasing element (6) in the form of an axial compression spring, especially a coil spring, which is supported to be fixed to the casing and at a spring action point (5.4) applies the pretensioning force to the clamping blade (5) in the pinching direction.

6. The volumetric pump according to one of the preceding claims, **characterized in that** the projection (4.1), at which the engaging surface (5.3) of the clamping blade (5) slips off, extends substantially in closing direction of the door (4), wherein the projection (4.1) undercuts the engaging surface (5.3) of the clamping blade (5) when the door (4) is closing and wherein the projection (4.1) continuously pivots the clamping blade (5) in the way of a ramp as the closing movement is proceeding.

7. The volumetric pump according to claim 6, **characterized in that** the projection (4.1) at which the engaging surface (5.3) of the clamping blade (5) slips off is in the form of an arcuate or pitch circle-shaped guiding contour at which the engaging surface (5.3) of the clamping blade (5) slips off such that the clamping blade (5) is caused to be lifted by forced guiding and thus the flexible tube (2) is caused to be released.

8. The volumetric pump according to one of the preceding claims, **characterized by** an inserting portion (7) which is formed by a casing portion adjacent to the clamping jaw (3.1) and a clamp portion adjacent to the clamping surface (5.2) so that a flexible tube (2) can be inserted therein at a position adjacent to the clamping surface (5.2) between the casing (3) and the clamping blade (5) and which is adjusted by design in such manner that a flexible tube (2) inserted therein is held positively and/or by application of a pretensioning force in a state released for flow.

9. The volumetric pump according to claim 8, **characterized in that** the door or flap (4) includes a projection-type or rib-type plug-in portion (4.2) which, when the door or flap (4) is closed, pushes a flexible tube (2) inserted in the inserting portion (7) between the clamping jaw (3.1) and the clamping surface (5.2).

10. The volumetric pump according to one of the preceding claims, **characterized in that** the pump casing (3), in particular in a lower edge portion of the accommodating chamber, forms a stop (3.2) against which the clamping blade (5) bears, when the flap or door (4) is opened, with a stop portion (5.5) by application of pretensioning force and which by design is adjusted to the clamping blade (5.5) and the clamping jaw (3.1) so that, with the clamping blade (5) bearing thereon, it defines a gap between the clamping surface (5.2) and the clamping jaw (3.1).

11. A method of controlling the flow through a tube (2) arranged on or in a pump casing (3) of a volumetric pump according to one of the preceding claims and adapted to be closed by means of a door or flap (4), thepump casing having a clamping blade (5) articulated on it in a relatively movable manner and which is configured to interact with a clamping jaw (3.1) fixed to the pump casing so as to pinch off the tube (2) in a sealing manner,
**characterized by** the steps of:
- inserting the tube (2) at a predetermined position on or in the pump casing (3);
- closing the pump casing (3) by means of the door (4) and at the same time moving the tube clamp (5) into the opening position by forced guiding and moving the tube (2) onto a clamping surface of the clamp due to the movement of the door (4);
- opening the door (4) and at the same time automatically closing the tube (2) by the tube clamp (5).

## Revendications

1. Pompe volumétrique avec un dispositif de serrage de tuyau (1) et
un carter de pompe (3), auquel est articulé un clapet ou une porte (4) pour la fermeture d'un espace de réception pour un tuyau flexible (2), dans lequel un collier de serrage (5) est agencé, collier qui est formé afin de libérer le tuyau (2) introduit dans celui-ci en cas de clapet ou de porte (4) fermé et de le serrer de manière étanche en cas de clapet ou de porte (4) ouvert,
**caractérisée en ce que**
le collier de serrage (5) présente une lame de serrage (5) précontrainte par ressort dans le sens de serrage ainsi que logée de manière mobile au niveau du carter de pompe (3), lame qui forme au niveau d'une section une surface de serrage (5.2) interagissant avec une mâchoire de serrage (3.1) fixée au carter de pompe, et qui forme au niveau d'une autre section une surface d'engagement (5.3), au niveau de laquelle une saillie (4.1) prévue au niveau du clapet ou de la porte (4) glisse lors d'un processus de fermeture par un mouvement de pivotement du clapet ou de la porte (4) de telle manière une force de déplacement soit appliquée ici sur la lame de serrage (5) à l'encontre de la précontrainte par ressort.

2. Pompe volumétrique selon la revendication 1, **caractérisée en ce que** la lame de serrage (5) est logée, en particulier au niveau d'une section d'extrémité de celle-ci, par le biais d'un axe de serrage (5.1) de manière rotative au niveau du carter de pompe (3).

3. Pompe volumétrique selon la revendication 1 ou 2, **caractérisée en ce que** la surface de serrage (5.2) et/ou la surface d'engagement (5.3) est agencée au niveau d'au moins une section d'extrémité, de préférence au niveau d'au moins une section d'extrémité opposée à l'axe de serrage (5.1), le plus préférentiellement au niveau de deux sections d'extrémité s'étendant en forme de branche en s'éloignant de l'axe de serrage (5.1), de la lame de serrage (5).

4. Pompe volumétrique selon l'une quelconque des revendications 1 à 3, **caractérisée en ce que** la précontrainte par ressort est conçue de telle manière qu'un tuyau (2) flexible introduit entre la surface de serrage (5.2) de la lame de serrage (5) et la mâchoire de serrage (3.1) du boîtier (3) en cas de clapet ou de porte (4) ouvert suite à la précontrainte par ressort est serré de manière étanche en surmontant son élasticité interne.

5. Pompe volumétrique selon l'une quelconque des revendications précédentes, **caractérisée par** un élément de précontrainte (6) réalisé comme un ressort de compression axial, en particulier comme ressort hélicoïdal, qui est logé de manière fixe au boîtier et applique au niveau d'un point d'action de ressort (5.4) la force de précontrainte dans le sens de serrage sur la lame de serrage (5).

6. Pompe volumétrique selon l'une quelconque des revendications précédentes, **caractérisée en ce que** la saillie (4.1), au niveau de laquelle la surface d'engagement (5.3) de la lame de serrage (5) glisse, s'étend sensiblement dans le sens de fermeture de la porte (4), vient en prise sous la surface d'engagement (5.3) de la lame de serrage (5) lors de la fermeture de la porte (4) et fait pivoter en continu la lame de serrage (5) à la manière d'une rampe avec un mouvement de fermeture croissant.

7. Pompe volumétrique selon la revendication 6, **caractérisée en ce que** la saillie (4.1), au niveau de laquelle la surface d'engagement (5.3) de la lame de serrage (5) glisse, est réalisée comme un contour de guidage en forme d'arc ou de cercle partiel, au niveau duquel la surface d'engagement (5.3) glisse lors d'une fermeture du clapet ou de la porte (4) de telle manière qu'un levage guidé à force de la lame de serrage (5) et ainsi une libération du tuyau flexible (2) soit provoquée.

8. Pompe volumétrique selon l'une quelconque des revendications précédentes, **caractérisée par** une section de placement (7) qui est formée par une section de carter contiguë à la mâchoire de serrage (3.1) et une section de serrage contiguë à la surface de serrage (5.2), de sorte qu'un tuyau flexible (2) puisse y être inséré dans la position contiguë à la surface de serrage (5.2) entre le carter (3) et la lame de serrage (5) et qui est adapté de manière constructive de telle manière qu'un tuyau (2) flexible qui y est inséré soit maintenu à complémentarité de formes et/ou en subissant une force de précontrainte dans un état libéré pour l'écoulement.

9. Pompe volumétrique selon la revendication 8, **caractérisée en ce que** la porte ou le clapet (4) présente une section d'insertion (4.2) de type à saillie ou nervure qui pousse, lors d'une fermeture de la porte ou du clapet (4), un tuyau (2) flexible introduit dans la section de placement (7) entre la mâchoire de serrage (3.1) et la surface de serrage (5.2).

10. Pompe volumétrique selon l'une quelconque des revendications précédentes, **caractérisée en ce que** le carter de pompe (3) forme, en particulier dans une section de bord inférieure de l'espace de réception, une butée (3.2), contre laquelle la lame de serrage (5) repose en cas de clapet ou de porte (4) ouvert avec une section de butée (5.5) subissant une force de précontrainte, et qui est adaptée de manière constructive à la lame de serrage (5) et à la mâchoire de serrage (3.1) de telle manière qu'elle définisse une fente entre la surface de serrage (5.2) et la mâchoire de serrage (3.1) en cas de lame de serrage (5) reposante.

11. Procédé de commande du flux traversant un tuyau (2) qui est agencé au niveau de ou dans un carter de pompe (3) refermable au moyen d'une porte ou d'un clapet (4) d'une pompe volumétrique selon l'une quelconque des revendications précédentes, à laquelle une lame de serrage (5) est articulée selon un mouvement relatif qui est formée afin d'interagir avec une mâchoire de serrage (3.1) fixée au carter de pompe pour serrer le tuyau (2) de manière étanche.
**caractérisé par** les étapes suivantes :
- l'introduction du tuyau (2) dans une position prédéterminée au niveau de ou dans le carter de pompe (3) ;
- la fermeture du carter de pompe (3) au moyen de la porte (4) et le déplacement simultané guidé de manière forcée du collier de serrage (5) dans la position d'ouverture et du tuyau (2) sur une surface de serrage du collier suite au mouvement de la porte (4) ;
- l'ouverture de la porte (4) et la fermeture simultanée autoactionnée du tuyau (2) par le collier de serrage (5).
